# EUROPÄISCHE PATENTSCHRIFT

(11) **EP 0 300 168 B1**
(45) Veröffentlichungstag und Bekanntmachung des Hinweises auf die Patenterteilung: **13.01.1993**
(21) Anmeldenummer: 88108567.4
(22) Anmeldetag: 28.05.1988
(51) Int. Cl.: C12N 15/52, C12P 21/02, C12N 9/00

(54) **Verfahren zur Synthese von Glutathion in Hefen**
Process for the glutathione synthesis in yeast
Procédé de synthèse de glutation dans la levure

(30) Priorität: 23.07.1987 DE 3724345
(43) Veröffentlichungstag der Anmeldung: 25.01.1989
(73) Patentinhaber: HÜLS AKTIENGESELLSCHAFT, 45764 Marl (DE)
(72) Erfinder: Lang-Hinrichs, Christine, Dr., D-1000 Berlin 28 (DE); Stahl, Ulf, Prof. Dr., D-1000 Berlin 28 (DE)

(56) Entgegenhaltungen:
- EP-A- 0 107 400
- CHEMICAL ABSTRACTS, Band 104, Nr. 9, 3. März 1986, Seite 202, Zusammenfassung Nr. 63390p, Columbus, Ohio, US; & JP-A-60 180 592 (M. KIMURA et al.) 14-09-1985
- CHEMICAL ABSTRACTS, Band 105, Nr. 21, November 1986, Seite 197, Zusammenfassung Nr. 185228h, Columbus, Ohio, US; K. WATANABE et al.: "Glutathione production by Escherichia coli cells with hybrid plasmid containing tandemly polymerized genes for glutathione sythetase", & APPL. MICROBIOL. BIOTECHNOL. 1986, 24(5), 375-8
- CHEMICAL ABSTRACTS, Band 105, Nr. 9, 1. September 1986, Seite 533, Zusammenfassung Nr. 77581j, Columbus, Ohio, US; & JP-A-61 52 299 (KANEGAFUCHI CHEMICAL INDUSTRY CO. LTD) 14-03-1986
- NUCLEIC ACIDS RESEARCH, Band 14, Nr. 11, Juni 1986, Seiten 4393-4400, IRL Press Ltd, Oxford, GB; K. WATANABE et al.: "The nucleotide sequence of the gene for gamma-glutamylcysteine synthetase of Escherichia coli"
- CHEMICAL ABSTRACTS, Band 100, Nr. 7, 13. Februar 1984, Seite 146, Zusammenfassung Nr. 46299r, Columbus, Ohio, US; H. GUSHIMA et al.: "Construction of glutathione-producing strains of Escherichia coli B by recombinant DNA techniques", & J. APPL. BIOCHEM. 1983, 5(1-2), 43-52

## Beschreibung

Die Erfindung betrifft ein gentechnisches Verfahren zur Synthese von Glutathion in Hefen. Die Synthese erfolgt dabei unter Einsatz von multiplen Glutathion-Synthesegenen.

Glutathion ist ein Tripeptid der Struktur gamma-Glutamyl-L-cysteinyl-glycin, das eine bedeutende Rolle als Cofaktor verschiedener Enzyme und als Schutz der Zellen gegen oxidative Schädigung durch toxische Verbindungen, reaktive Sauerstoff-Derivate und Strahlung und generell in der Inaktivierung von zellschädlichen Substanzen besitzt. Der Einsatz dieses Stoffes als Therapeutikum, z. B. zur vorbeugenden Behandlung von Leber-Erkrankungen, macht eine biotechnologische Synthese wünschenswert.
Glutathion wird intrazellulär durch die aufeinanderfolgende Aktion der Enzyme gamma-Glutamylcysteinsynthetase und Glutathionsynthetase aus den Aminosäuren L-Glutaminsäure, L-Cystein und Glycin gebildet.

Die Reaktionsgleichungen lauten: 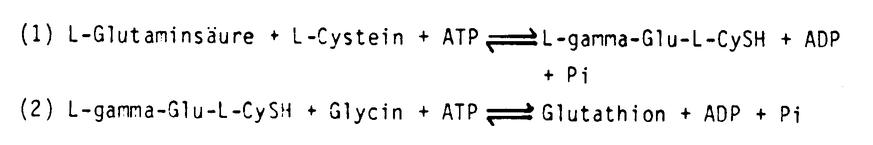
- ATP =: Adenosintriphosphat
- ADP =: Adenosindiphosphat
- Pi =: anorganisches Phosphat
Für die Synthese der Enzyme sind Enzym-kodierende Gene verantwortlich. Für das Enzym gamma-Glutamylcysteinsynthetase ist es das GSH-I-Gen, für das Enzym Glutathionsynthetase ist es das GSH-II-Gen.

Die Isolation von Glutathion erfolgt üblicherweise aus Zellen von Rakterien oder Hefen, bei denen durch ein besonderes Anzuchtverfahren oder eine besondere Zusammensetzung des Nährmediums oder durch mutative Veränderung der verwendeten Stämme eine Anreicherung des Peptids erreicht wird. Derartige Verfahren werden beispielsweise in US-PS 4 582 801 und US-PS 4 596 775 genannt.

Ein von Watanabe, Appl. Microbiol. Biotechnol. 24, 375-378 (1986), beschriebenes Verfahren verwendet die beiden Glutathionsynthese-Gene zur Produktion von Glutathion in Bakterien (Escherichia coli). Dabei steigt die spezifische enzymatische Aktivität des Glutathion-synthetisierenden Systems bis auf das 40fache des Elternstammes.

Weiterhin wird in JP-PS 86/52 299 der Einsatz des GSH-1-Gens in Hefezellen beschrieben. Dabei wird eine Erhöhung der Glutathion-Produktion um 30 % beobachtet.

Die Nukleotidsequenz der beiden Escherichia coli-Gene, die für die an der Glutathion-Synthese beteiligten Enzyme kodieren, wurde von Watanabe, Nucleic Acids Research 14, 4393-4400 (1986), bzw. von Gushima, Nucleic Acids Research 12, 9299-9307 (1984), bestimmt.

Die bekannten Verfahren gewähren für Hefe noch keinen gezielten Eingriff in die bekannten Synthesewege des Glutathions und damit noch keine gesteuerte Glutathionsynthese in Hefe. Die Verwendung des Gens GSH II und ein kombinierter Einsatz der beiden Gene GSH I und GSH II sind nicht bekannt.

Aufgabe der vorliegenden Erfindung ist es, ein verbessertes Verfahren zur Synthese von Glutathion in Hefen bereitzustellen.

Die Aufgabe wird erfindungsgemäß dadurch gelöst, daß man das GSH-II-Gen oder eine Kombination aus GSH-II-Gen und GSH-I-Gen in die Hefen einführt.

Die Enzym-kodierenden Gene GSH I und GSH II, die für das Bakterium Escherichia coli im Detail bekannt sind und in einer Vielzahl weiterer Organismen vorkommen, werden mit Hilfe der üblichen Methoden der Molekularbiologie aus einem beliebigen Organismus isoliert und auf einem Plasmid kloniert. Dieses besteht entweder nur aus Hefe-DNA (Desoxynucleinsäure) oder aus zusätzlich anderen DNA-Teilen, es trägt einen selektiven Marker (ein bakterielles oder eukaryotisches Gen) und einen DNA-Bereich, der die autonome Replikation des Plasmids in Hefe bewerkstelligt, oder einen zum Hefe-Genom homologen Anteil, der die Integration des Plasmids ins chromosomale Genom der Hefe bewirkt. Dabei werden die isolierten GSH-I- und GSH-II-Gene entweder direkt nach Isolation aus dem Genom des jeweiligen Organismus oder nach Kopplung mit für Hefe passenden Erkennungssignalen für die Transkription oder Translation (Promotoren, Terminatoren, Ribosomenbindestellen) in die so erstellten Plasmide eingebaut.

Die so konstruierten Plasmide zeichnen sich dadurch aus, daß sie entweder das GSH-II-Gen oder das GSH-II-Gen und das GSH-I-Gen tragen.

Die Plasmide werden unter Anwendung von bekannten Methoden in einen gewünschten Hefe-Wirtsstamm eingeführt. Derartige Methoden sind beispielsweise die Protoplasten-Transformation und die LithiumacetatTransformation.

Die Hefe-Wirtsstämme können sowohl Labor- als auch Industriestämme sein. Man kann Hefen wie Bäcker-, Bier- oder Weinhefen einsetzen. Vorzugsweise werden jedoch Bäckerhefen, im besonderen Saccharomyces cerevisiae, verwendet.

Im Vergleich zu den unveränderten Hefestämmen sind diese gentechnisch veränderten Stämme in der Lage, die Glutathion-synthetisierenden Gene in erhöhtem Maße zu transkribieren, von diesen Transkripten die Glutathion-synthetisierenden Proteine zu bilden, eine erhöhte Menge an Glutathion zu synthetisieren und diese anzureichern.

Auf diese Weise kann die Produktion von Glutathion durch Hefen um mehr als das 6fache des Elternstammes gesteigert werden.

Die Glutathion-Syntheseeigenschaften der Hefewirtsstämme sind dabei keine zufälligen Resultate von ungezielten Mutationen. Es sind genau definierte Eigenschaften. Dabei erlaubt die Untersuchung der mRNA-Zwischenprodukte und der Enzymaktivitäten eine gezielte Optimierung die ser Eigenschaften durch Austausch von Regulationssequenzen, durch Variation der Kopienzahl der Gene, durch Aktivierung der Gene mittels äußerer Faktoren (regulierbare Promotoren). Die Optimierung kann systematisch den Bedürfnissen des Wirtsstammes und/oder den Produktionsbedingungen angepaßt werden.

Gegenüber Escherichia coli haben die Hefen den Vorteil, daß bei der Isolierung von Glutathion keine toxischen Proteine abgetrennt werden müssen.

Die folgenden Beispiele sollen die Erfindung erläutern.

Herstellung eines Plasmids, das das GSH-I-Gen enthält:
Das Plasmid pPT2a, das aus einem bakteriellen Anteil (pBR322) und einem Hefeanteil (2 µm-Replikationsursprung und 2 µm-Able-Promotor (vgl. Sutton und Broach, Molecular and Cellular Biology 5 (1985), 2770-2780) und Trpl-Terminator) besteht, wird mit Hilfe des Restriktionsenzyms Eco R I an 2 µm-Able-Promotor geöffnet. Die dabei entstehenden Einzelstrangenden werden nun mit Hilfe der Klenow-DNA-Polymerase aufgefüllt. Aus dem Plasmid pGSI15 wird das Escherichia coli GSH-I-Gen mit Hilfe der Restriktionsenzyme Eco R I und Nde I herausgeschnitten, mittels Agarose-Gelelektrophorese abgetrennt und mittels Elektroelution isoliert. Die überstehenden Einzelstrangenden werden mit Klenow-DNA-Polymerase aufgefüllt, und das GSH-I-Gen wird mittels T4-DNA-Ligase mit dem Plasmid pPT2a verknüpft. Die ligierte DNA wird in den Escherichia coli-Stamm SF8 transformiert. Die Plasmide von Ampicillin-resistenten Einzelkolonien werden isoliert. Dabei wird das Plasmid pPTIN6 durch Agarose-Gelelektrophorese und Restriktionsanalyse identifiziert.

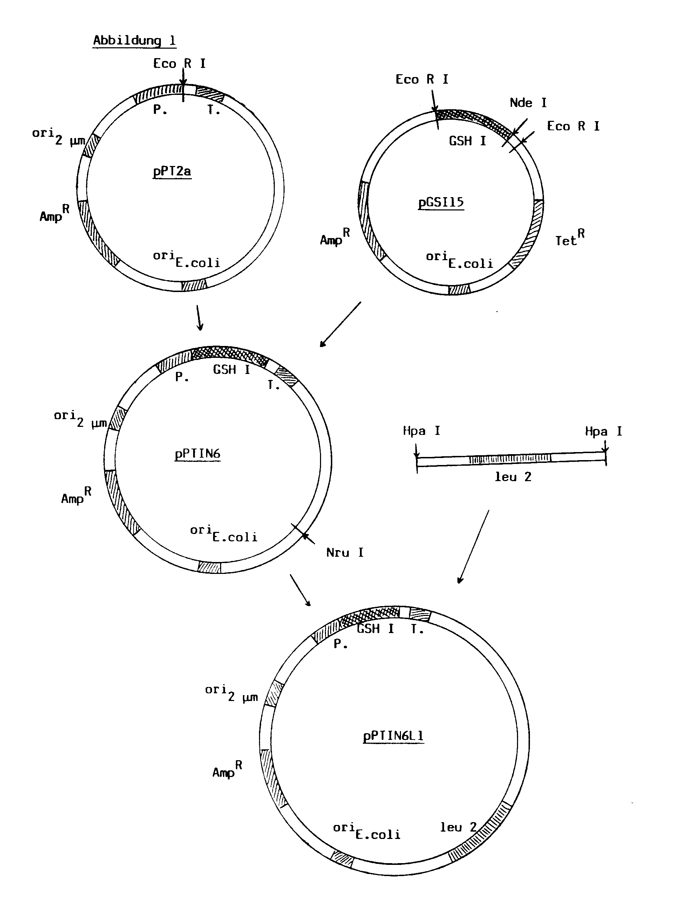

Das Plasmid pPTIN6 wird mit dem Enzym Nru I restringiert und mit einem Teil der chromosomalen Hefe-DNA, der das Leucin-2-Gen trägt und der mit dem Enzym Hpa I geschnitten wurde, ligiert, um das Plasmid pPTIN6L1 zu ergeben (vgl. Abbildung 1).

### Beispiel 1

Aus dem Plasmid pGS201 wird mittels Bam H I- und unvollständiger Eco R V-Restriktion ein DNA-Fragment abgetrennt, das das Escherichia coli GSH-II-Gen trägt. Das entsprechende DNA-Fragment wird mittels Elektroelution nach Auftrennung auf einem Agarose-Gel isoliert, worauf die Einzelstrangenden mit Hilfe der Klenow-DNA-Polymerase aufgefüllt werden. Das so behandelte DNA-Fragment wird mit Hilfe der T4-DNA-Ligase mit dem Plasmid pPT2a, das wie in Beispiel 1 behandelt wurde, verknüpft. Die ligierte DNA wird in den Escherichia coli-Stamm SF8 transformiert. Aus Ampicillin-resistenten Einzelkolonien wird die DNA isoliert. Das Plasmid pPTEII75 wird mit Hilfe der Agarose-Gelelektrophorese und Restriktionsanalyse identifiziert.

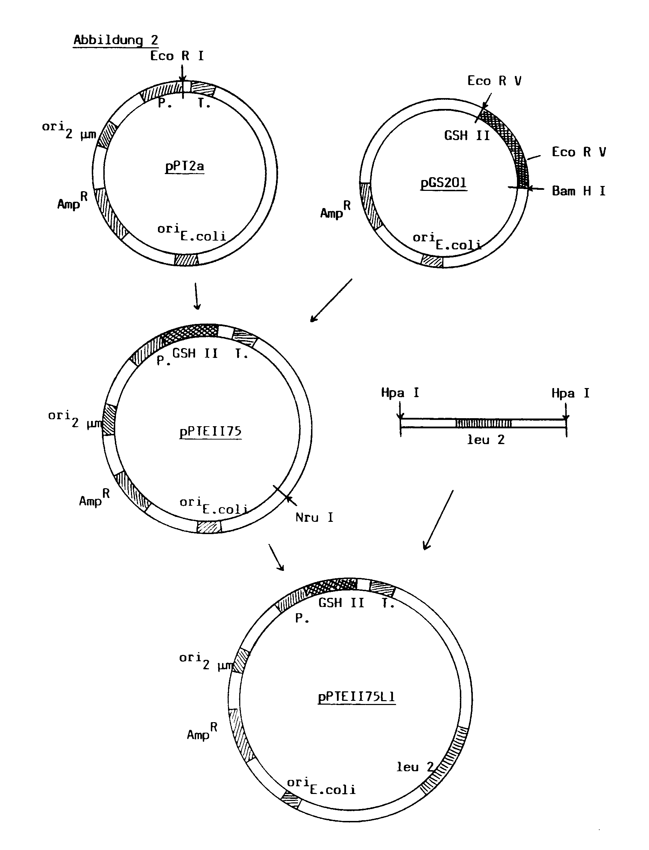

Dieses Plasmid wird mit dem Enzym Nru I linearisiert und mit einem Teil der chromosomalen Hefe-DNA, der das Leucin-2-Gen trägt und mit dem Enzym Hpa I geschnitten wurde, ligiert, wobei man das Plasmid pPTEII75L1 erhält (vgl. Abbildung 2).

Das so konstruierte Plasmid wird in den Hefestamm Saccharomyces cerevisiae AH 22 (vgl. Hinnen u. a., Proceeding of the National Acadamy of Science, USA 75 (1978), 1929-1933) eingeführt, der eine leu-2-Auxotrophie trägt. Anschließend wird der Leucin-prototrophe transformierte Stamm selektiert.
Die Glutathionsynthese-Aktivität des Transformanten wird mittels eines enzymatischen Tests (in vitro-Glutathion-Synthese mit anschließender Glyoxalase-Bestimmung), der Glutathion-Gehalt mittels Glyoxalase-Bestimmung ermittelt.

Der transformierte Stamm zeigt eine Glutathionsynthese-Aktivität von 0,5 µmol/h . mg Protein. Im Vergleich dazu besitzt der Ausgangsstamm AH 22 eine Aktivität von 0,1 µmol/h . mg Protein. Der Glutathion-Gehalt des transformierten Stammes beträgt 1,1 % der Trockensubstanz, der des Ausgangsstammes 0,5 % der Trockensubstanz.

### Beispiel 2

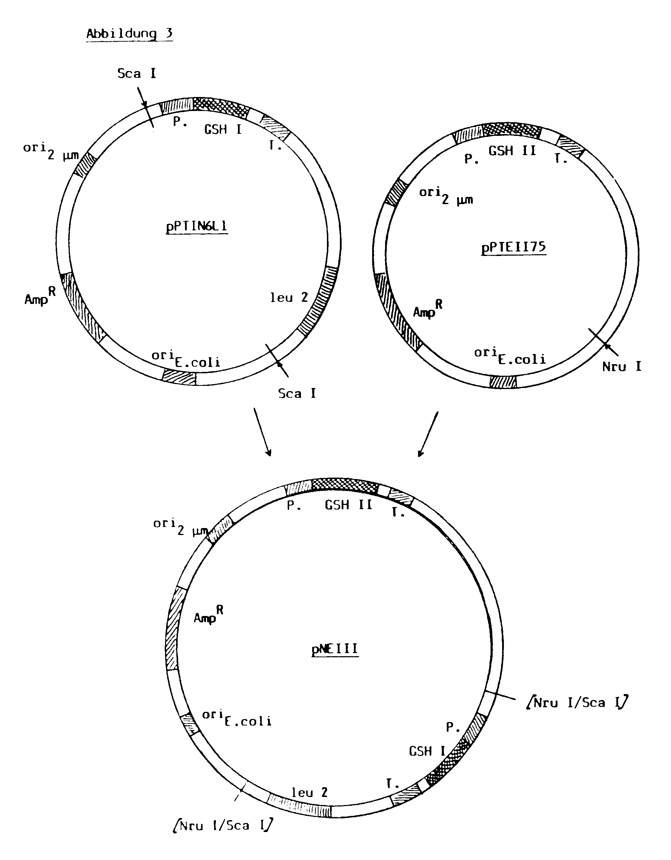

Das Plasmid pPTIN6L1, das das GSH-I-Gen enthält, wird mit dem Enzym Sca I ge schnitten und mit dem mit dem Enzym Nru I geschnittenen Plasmid pPTEII75 (siehe Beispiel 1) ligiert. Das Ligationsgemisch wird in den Escherichia coli-Stamm SF8 transformiert. Die DNA aus Leucin-auxotrophen und Ampicillin-resistenten Kolonien wird isoliert. Durch Restriktionsanalyse und Agarose-Gelelektrophorese wird das Plasmid pNEIII identifiziert (vgl. Abbildung 3).

Dieses Plasmid wird in Saccharomyces cerevisiae AH22 transformiert. Der transformierte Stamm hat eine Glutathionsynthese-Aktivität von 0,7 µmol/h . mg Protein. Der Glutathion-Gehalt des transformierten Stammes beträgt 1,6 % der Trockensubstanz.

### Beispiel 3

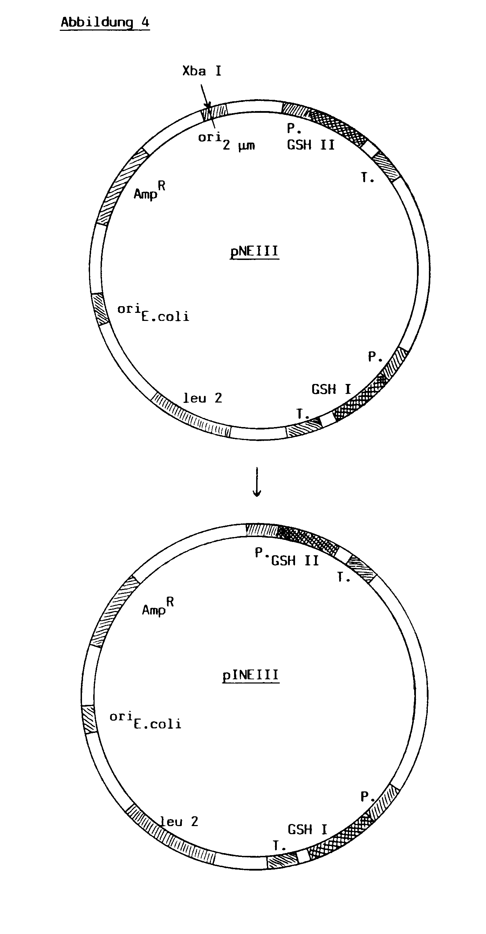

Das Plasmid pNEIII (siehe Beispiel 2) wird mit dem Restriktionsenzym Xba I linearisiert und mit der Exonuclease Ba131 so lange inkubiert, bis 200 bis 300 Basenpaare der Sequenz zu beiden Seiten der Xba I-Schnittstelle entfernt sind. Daraufhin wird das Plasmid mit dem Enzym T4-DNA-Ligase behandelt und in den Escherichia coli-Stamm SF8 transformiert. Durch DNA-Isolation aus Ampicillin-resistenten Kolonien und durch Restriktionsanalyse wird das Plasmid pINEIII identifiziert, bei dem der Replikationsursprung der 2 µm-DNA deletiert worden ist (vgl. Abbildung 4). Dieses Plasmid kann nun bei Transformation einer Hefezelle durch integrative Transformation in deren Chromosom eingebaut werden.

Die Einführung dieses Plasmids in Saccharomyces cerevisiae AH22 ergibt transformierte Kolonien, die eine Kopie des Plasmids im Genom integriert tragen. Der Glutathion-Gehalt des Transformanten liegt bei 1,8 % der Trockensubstanz.

### Abkürzungen in den Abbildungen 1 bis 4:

- Amp^{R}: Gen-kodierend für Ampicillin-Resistenz
- leu 2: Leucin-2-Gen von Saccharomyces cerevisiae
- ori_{E.coli}: Replikationsursprung in Escherichia coli
- ori_{2 µm}: Replikationsursprung der 2 µm-DNA, aktiv in Saccharomyces cerevisiae
- Tet^{R}: Gen-kodierend für die Tetracyclin-Resistenz
- Bam H I: Restriktionsendonukleasen
- Eco R I: "
- Eco R V: "
- Hpa I: "
- Nde I: "
- Nru I: "
- Sca I: "
- Xba I: "
- P.: Promotor
- T.: Terminator

## Patentansprüche

1. Verfahren zur Synthese von Glutathion in Hefen,
dadurch gekennzeichnet,
daß man das Enzym-kodierende Gen für das Enzym Glutathionsynthetase oder die Enzym-kodierenden Gene für die Enzyme gamma-Glutamylcysteinsynthetase und Glutathionsynthetase in die Hefen einführt.

2. Verfahren nach Anspruch 1,
dadurch gekennzeichnet,
daß man die Enzym-kodierenden Gene auf einem Plasmid kloniert in die Hefen einführt.

3. Verfahren nach Anspruch 2,
dadurch gekennzeichnet,
daß man Hefe-DNA-enthaltende Plasmide einsetzt.

4. Verfahren nach Anspruch 1,
dadurch gekennzeichnet,
daß man die Gene in Bäckerhefen einführt.

5. Verfahren nach Anspruch 4,
dadurch gekennzeichnet,
daß man die Gene in Saccharomyces cerevisiae einführt.

## Revendications

1. Procédé de synthèse de glutathion dans des levures,
caractérisé par le fait que l'on introduit dans les levures le gène codant l'enzyme glutathion-synthétase ou alors les gènes codant les enzymes gamma-glutamyl-cystéine-synthétase et glutathion-synthétase.

2. Procédé selon la revendication 1,
caractérisé par le fait que l'on introduit dans les levures, clonés sur un plasmide, les gènes codant les enzymes.

3. Procédé selon la revendication 2,
caractérisé par le fait que l'on utilise des plasmides contenant de l'ADN de levure.

4. Procédé selon la revendication 1,
caractérisé par le fait que l'on introduit les gènes dans de la levure de boulanger.

5. Procédé selon la revendication 4,
caractérisé par le fait que l'on introduit les gènes dans Saccharomyces cerevisiae.

## Claims

1. A process for the synthesis of glutathione in yeasts, characterised in that the enzyme-encoding gene for the enzyme glutathione synthetase or the enzyme-encoding genes for the enzymes gamma-glutamylcysteine synthetase and glutathione synthetase are introduced into the yeasts.

2. A process according to claim 1, characterised in that the enzyme-coding genes are introduced cloned on a plasmid into the yeasts.

3. A process according to claim 2, characterised in that plasmids containing yeast DNA are employed.

4. A process according to claim 1, characterised in that the genes are introduced into baker's yeasts.

5. A process according to claim 4, characterised in that the genes are introduced into Saccharomyces cerevisiae.
